# EUROPEAN PATENT SPECIFICATION

(11) **EP 1 037 612 B1**
(45) Date of publication and mention of the grant of the patent: **25.02.2004**
(21) Application number: 98966322.4
(22) Date of filing: 14.12.1998
(51) Int. Cl.: A61K 9/14, A61K 31/35

(54) **PHARMACEUTICAL COMPOSITIONS CONTAINING MICRONIZED BICYCLIC DRUGS**
MIKRONISIERTE BICYCLISCHE ARZNEISTOFFE ENTHALTENDE PHARMAZEUTISCHE ZUSAMMENSETZUNGEN
PREPARATIONS PHARMACEUTIQUES CONTENANT DES MEDICAMENTS BICYCLIQUES MICRONISES

(30) Priority: 16.12.1997 GB 9726543
(43) Date of publication of application: 27.09.2000
(60) Divisional application: 03076016.9
(73) Proprietor: SmithKline Beecham plc, Brentford, Middlesex TW8 9GS (GB)
(72) Inventor: DUMPLETON, David Robert, SmithKline Beecham Phar., Harlow, Essex CM19 5AW (GB); HOLLAND, Simon Joseph, SmithKline Beecham Pharm., Harlow, Essex CM19 5AW (GB); KNIGHT, Wendy Anne, SmithKline Beecham Pharm., Harlow, Essex CM19 5AW (GB); LEONARD, Graham Stanley, SmithKline Beecham Phar., Harlow, Essex CM19 5AW (GB)
(74) Representative: West, Vivien
(86) International application number: PCT/EP1998/008156
(87) International publication number: WO 1999/030687

(56) References cited:
- EP-A- 0 499 299
- WO-A-94/13657
- WO-A-95/34545
- US-A- 5 518 738
- US-A- 5 534 270

## Description

This invention is concerned with novel compositions of compounds having therapeutic activity in particulate form. especially particulate compositions of sparingly water soluble compounds, with enhanced bio-availability; and to the use of such compositions in therapy.

EP-A-0 126 311 and EP-A-0 139 992 disclose benzopyran derivatives which have antihypertensive activity. WO 92/22293, WO 94/13657 and WO 95/34545 disclose that the benzopyran compounds also have anxiolytic and anticonvulsant activity and disclose further benzopyran derivatives and related pyridopyran derivatives having similar activities. Unpublished International Patent Application No PCT/EP97/05168 discloses additional utilities for these compounds, including the treatment of neuropathic pain.

It is known to subject pharmaceutically active compounds to milling procedures to obtain a particle size appropriate for tablet formation and for other formulation types. Air jet milling and fluid energy milling (micronising) have been favoured because of the reduced risk from introducing contamination from mill materials. However, wet milling processes have been proposed for preparation of finely divided particles for pharmaceutical use, for example US-A-4 540 602 and EP-A-0 499 299. The latter discloses a wet milling procedure to produce particles of a crystalline drug substance having a surface modifier adsorbed on the surface thereof in an amount sufficient to maintain an effective average particle size of less than about 400 nm. This particulate composition is said to provide improved bioavailability for poorly water soluble compounds.

The present invention is based on the finding that the benzopyran and pyridopyran compounds mentioned above have enhanced bio-availability in certain compositions of controlled particle size. This invention also provides a method of producing such compositions in a reproducible manner.

According to the present invention there is provided a composition comprising a compound of Formula I or Formula II below in particulate form having a monomodal particle size distribution such that the median value of the volume mean diameter is within the range of from 350 to 700 nm, typically with no more than 10% of the particles having a volume diameter of 280 nm or below, and no less than 90% of the particles having a volume diameter of 2000 nm or below.

A composition of this invention may be in the form of an aqueous dispersion of the particles of a compound of Formula I or Formula II; typically the product of a wet milling operation. A composition of this invention may also be a powder comprising particles of a compound of Formula I or Formula 11; typically the product of a spray drying or spray granulation procedure.

Compounds used in this invention are as follows: 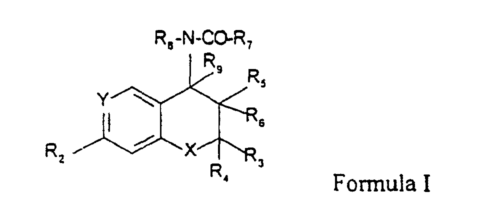 wherein:
either Y is N and R₂ is hydrogen, or Y is C-R₁
where:
either one of R₁ and R₂ is hydrogen and the other is selected from the class of hydrogen, C₃₋₈ cycloalkyl, C₁₋₆ alkyl optionally interrupted by oxygen or substituted by hydroxy, C₁₋₆ alkoxy or substituted aminocarbonyl, C₁₋₆ alkylcarbonyl, C₁₋₆ alkoxycarbonyl, C₁₋₆ alkylcarbonyloxy, C₁₋₆ alkoxy, nitro, cyano, halo, trifluoromethyl, CF₃S, or a group CF₃-A-, where A is -CF₂-, -CO-, -CH₂- or CH(OH), trifluoromethoxy, C₁₋₆ alkylsulphinyl, C₁₋₆ alkylsulphonyl, C₁₋₆ alkoxysulphinyl, C₁₋₆ alkoxysulphonyl, aryl, heteroaryl, arylcarbonyl, heteroarylcarbonyl, arylsulphinyl, heteroarylsulphinyl, arylsulphonyl, heteroarylsulphonyl in which any aromatic moiety is optionally substituted, C₁₋₆ alkylcarbonylamino, C₁₋₆ alkoxycarbonylamino, C₁₋₆ alkyl-thiocarbonyl, C₁₋₆ alkoxy-thiocarbonyl, C₁₋₆ alkyl-thiocarbonyloxy, 1-mercapto C₂₋₇ alkyl, formyl, or aminosulphinyl, aminosulphonyl or aminocarbonyl, any amino moiety being optionally substituted by one or two C₁₋₆ alkyl groups, or C₁₋₆ alkylsulphinylamino, C₁₋₆ alkylsulphonylamino,C₁₋₆ alkoxysulphinylamino or C₁₋₆ alkoxysulphonylamino, or ethylenyl terminally substituted by C₁₋₆ alkylcarbonyl, nitro or cyano, or -C(C₁₋₆ alkyl)NOH or -C(C₁₋₆ alkyl)NNH₂, or one of R₁ and R₂ is nitro, cyano or C₁₋₃ alkylcarbonyl and the other is methoxy or amino optionally substituted by one or two C₁₋₆ alkyl or by C₂₋₇ alkanoyl;
one of R₃ and R₄ is hydrogen or C₁₋₄ alkyl and the other is C₁₋₄ alkyl or R₃ and R₄ together are C₂₋₅ polymethylene;
R₅ is C₁₋₆ alkylcarbonyloxy, benzoyloxy, ONO₂, benzyloxy, phenyloxy or C₁₋₆ alkoxy and R₆ and R₉ are hydrogen or R₅ is hydroxy and R₆ is hydrogen or C₁₋₂ alkyl and R₉ is hydrogen;
R₇ is fluorophenyl;
R₈ is hydrogen or C₁₋₆ alkyl;
the R₈-N-CO-R₇ group being *trans* to the R₅ group;
and X is oxygen or NR₁₀ where R₁₀ is hydrogen or C₁₋₆ alkyl;
and 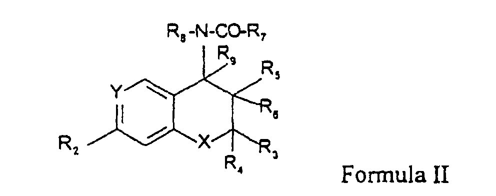 wherein:
either Y is N and R₂ is hydrogen, or Y is C-R₁
where:
either one of R₁ and R₂ is hydrogen and the other is selected from the class of hydrogen, C₃₋₈ cycloalkyl, C₁₋₆ alkyl optionally interrupted by oxygen or substituted by hydroxy, C₁₋₆ alkoxy or substituted aminocarbonyl, C₁₋₆ alkylcarbonyl, C₁₋₆ alkoxycarbonyl, C₁₋₆ alkylcarbonyloxy, C₁₋₆ alkoxy, nitro, cyano, halo, trifluoromethyl, CF₃S, or a group CF₃-A-, where A is -CF₂-, -CO-, -CH₂-, CH(OH), SO₂, SO, CH₂-O, or CONH, or a group CF₂H-A'- where A' is oxygen, sulphur, SO, SO₂, CF₂ or CFH; trifluoromethoxy, C₁₋₆ alkylsulphinyl, perfluoro C₂₋₆ alkylsulphonyl, C₁₋₆ alkylsulphonyl, C₁₋₆ alkoxysulphinyl, C₁₋₆ alkoxysulphonyl, aryl, heteroaryl, arylcarbonyl, heteroarylcarbonyl, phosphono, arylcarbonyloxy, heteroarylcarbonyloxy, arylsulphinyl, heteroarylsulphinyl, arylsulphonyl, heteroarylsulphonyl in which any aromatic moiety is optionally substituted, C₁₋₆ alkylcarbonylamino, C₁₋₆ alkoxycarbonylamino, C₁₋₆ alkyl-thiocarbonyl, C₁₋₆ alkoxy-thiocarbonyl. C₁₋₆ alkyl-thiocarbonyloxy, 1-mercapto C₂₋₇ alkyl, formyl, or aminosulphinyl, aminosulphonyl or aminocarbonyl, any amino moiety being optionally substituted by one or two C₁₋₆ alkyl groups, or C₁₋₆ alkylsulphinylamino, C₁₋₆ alkylsulphonylamino, C₁₋₆ alkoxysulphinylamino or C₁₋₆ alkoxysulphonylamino, or ethylenyl terminally substituted by C₁₋₆ alkylcarbonyl, nitro or cyano, or -C(C₁₋₆ alkyl)NOH or -C(C₁₋₆ alkyl)NNH₂, or one of R₁ and R₂ is nitro, cyano or C₁₋₃ alkylcarbonyl and the other is methoxy or amino optionally substituted by one or two C₁₋₆ alkyl or by C₂₋₇ alkanoyl; or R₁ and R₂ together are -(CH₂)₄- or -CH = CH-CH = CH-, or form an optionally substituted triazole or oxadiazole ring;
one of R₃ and R₄ is hydrogen or C₁₋₄ alkyl and the other is C₁₋₄ alkyl, CF₃ or CH₂ X^{a} where X^{a} is fluoro, chloro, bromo, iodo, C₁₋₄ alkoxy, hydroxy, C₁₋₄ alkylcarbonyloxy, -S-C₁₋₄ alkyl, nitro, amino optionally substituted by one or two C₁₋₄ alkyl groups; cyano or C₁₋₄ alkoxycarbonyl or R₃ and R₄ together are C₂₋₅ polymethylene optionally substituted by C₁₋₄ alkyl;
R₅ is C₁₋₆ alkylcarbonyloxy, benzoyloxy, ONO₂, benzyloxy, phenyloxy or C₁₋₆ alkoxy and R₆ and R₉ are hydrogen or R₅ is hydroxy and R₆ is hydrogen or C₁₋₂ alkyl and R₉ is hydrogen;
R₇ is heteroaryl or phenyl; both of which are optionally substituted one or more times independently with a group or atom selected from chloro, fluoro, bromo, iodo, nitro, amino optionally substituted once or twice by C₁₋₄ alkyl, cyano, azido, C₁₋₄ alkyl, C₁₋₄ alkoxy, trifluoromethoxy and trifluoromethyl;
R₈ is hydrogen; C₁₋₆ alkyl, OR₉ or NHCOR₁₀ wherein R₉ is hydrogen, C₁₋₆ alkyl, formyl, C₁₋₆ alkanoyl, aroyl or aryl-C₁₋₆ alkyl and R₁₀ is hydrogen, C₁₋₆ alkyl, C₁₋₆ alkoxy, mono or di C₁₋₆ alkyl amino. amino, amino-C₁₋₆ alkyl, hydroxy-C₁₋₆ alkyl, halo-C₁₋₆ alkyl, C₁₋₆ acyloxy-C₁₋₆ alkyl, C₁₋₆ alkoxycarbonyl-C₁₋₆-alkyl, aryl or heteroaryl;
the R₈-N-CO-R₇ group being *cis* to the R₅ group;
and X is oxygen or NR₁₀ where R₁₀ is hydrogen or C₁₋₆ alkyl.

Compounds within the scope of Formula I and II are disclosed in EP-A-0 139 992, EP-A-0 126 311, WO 92/22293, WO 94/13657 and WO 95/34545. Reference is directed to these publications for information on the preparation and use of these compounds, on preferred substituents and substitution patterns, and on suitable individual compounds and their preparation.

Preferred compounds for use in this invention are *trans*-6-acetyl-4S-(4-fluorobenzoylamino)-3,4-dihydro-2,2-dimethyl-2H-1-benzopyran-3R-ol hereinafter Compound I (for preparation see Example 20 of WO 92/22293); and *cis*-6-acetyl-4S-(3-chloro-4-fluorobenzoylamino)-3,4-dihydro-2,2-dimethyl-2H-1-benzopyran-3S-ol hereinafter Compound 2 (for preparation see Example 17 of WO95/34545); and *trans*-6-acetyl-4S-(3,5-difluorobenzoylamino)-3,4-dihydro-2,2-dimethyl-2H-1-benzopyran-3R-ol (for preparation see Example 4 of WO95/34545).

The compounds used in this invention have a very low solubility in water. For example, Compound 1 has an aqueous solubility of 0.008 mg/ml, which is "virtually insoluble" under the USP classification.

The above-listed compounds may be used in the form of pharmaceutically acceptable solvates, especially hydrates. Before milling they may be in crystalline or amorphous form.

The compositions of this invention are most suitably prepared by wet milling compounds of Formula I and Formula II as an aqueous dispersion. For small scale processing the wet milling is preferably carried out using a two chamber bead mill with circulation of product between the chambers. To reduce contamination from mill materials, the milling medium is preferably ceramic beads of rare earth oxides and is carried out in chambers lined with or constructed from an abrasion-resistant polymer material such as nylon. For effective milling, the beads in one chamber are of a smaller diameter than the beads in the other chamber; effective results are obtainable with beads of 1.25 mm in one chamber and beads of 0.4 mm in the other chamber. The milling beads are preferably zirconia beads, especially beads made from yttria-stabilized zirconia powder. For larger scale processing a 5 chamber bead mill can be utilised. The product is processed in one pass through one, two, three, four or all five of the mills. For effective milling, the beads in the first chamber are between 0.65mm and 1.25mm. Beads in subsequent chambers are smaller than or of a similar size to the previous mill

To assist in further processing, that is preparation of pharmaceutical formulations for therapeutic use, such as tablets, injectable dispersions, etc., the wet milling of compounds of Formula I and Formula II preferably takes place in an aqueous medium including one or more excipients such as a soluble carrier suitable for spray drying, a surfactant to maintain the particles in suspension, and an anti-agglomeration agent effective after administration of a pharmaceutical formulation to a patient. An especially suitable excipient for spray-drying is mannitol but other carbohydrates such as sorbitol, lactose, lactitol and xylitol and starch may be used as carrier. Preferably sodium lauryl sulphate is used as surfactant, and cellulosic thickening agents such as hydroxypropyl methyl cellulose or hydroxyethyl cellulose may be used as anti-agglomeration agents.

In the aqueous medium to be subjected to the milling, the compound of Formula I or II may be present from about 10 to about 30% w/w. At 30% w/w and above there may be difficulties in maintaining a suspension of the compound of Formula I or II during milling. In practice, 20% w/w provides an effective compromise between the desire for a high throughput and short milling times.

The amount of the soluble carrier may vary from about 4 to about 15% w/w of the composition to be milled. Preferably the amount of the soluble carrier does not exceed 50% by weight of the amount of compound of Formula I or II to be processed. For a compound loading of about 20% w/w, an amount of soluble carrier from about 4 to 10% has been found to be effective and an amount of about 5% w/w is preferred.

The amount of surfactant may be varied from about 0.1 to about 0.4% w/w of the aqueous medium. Preferably it is present at about 1% by weight of the compound to be processed.

The amount of the anti-agglomeration agent is typically from about 1% w/w to about 2% w/w of the aqueous medium. An amount of about 1.5% w/w is preferred.

In a preferred embodiment of the invention, the median volume diameter is in the range of 400 to 600 nm, especially 450 to 550 nm. In this median range, effective compositions are obtained when 10% of particles have a volume diameter of 260 nm or below and 90% of particles have a volume diameter of 1450 nm or below.

Using the milling beads and aqueous carrier system described above, a composition having the preferred particle size distribution may be obtained surprisingly quickly, for example after milling for about 30 minutes on a small scale mill using product recirculation. Increasing the milling time, for example to about 1 hour, enables the largest particles to be reduced so that at least 90% of particles have a volume diameter of less than 1000 nm. However, the effect on the median value is marginal so longer milling times are not cost effective. Similarly, for large scale processing, batch sizes of up to 200Kg for a compound loading of about 20% w/w, can be processed surprisingly quickly, with the preferred particle size distribution obtained after one pass of product over a 70 minute period.
The aqueous dispersion obtained from the milling process may be used directly as a therapeutic agent if prepared under conditions of appropriate hygiene. Using water and other components which meet Ph Eur standards, compositions can be obtained which can be stored for up to one month at 5°C without bacterial growth being detected. However, for the preparation of formulations for use in human therapy, it is preferred that the aqueous dispersion is converted to a dried powder. This is carried out most suitably by spray drying, typically collecting the product from the dryer using a cyclone separator. By including the excipients mentioned above in the aqueous medium for milling, a powder composition containing the particulate compounds of Formula I and Formula II is obtainable as a composition which has good flowability and is suitable for incorporation into a tablet formulation or a powder formulation for capsules.

Compounds of Formula I and II have hitherto been formed into tablets by first micronising the drug substance and then wet granulating the micronised drug with the tablet excipients. The spray dried powder produced in this invention can be formed into tablets by direct compression. Surprisingly it has been found that the milling time to achieve the desired particle size distribution is substantially the same whether the starting material is micronised or unmicronised. The ability to commence the bead milling with unmicronised drug substance as obtained from the primary manufacturing process makes the present milling process especially cost-effective. The wet cake from manufacture is washed and tray dried and is then ready for bead milling. Alternatively, for certain compounds it may be possible for the wet cake to be washed and resuspended ready for bead milling, thus eliminating an expensive drying stage.

The compounds of Formula I and II are believed to be useful in the treatment and/or prophylaxis of anxiety, mania, depression, panic disorders and/or aggression, disorders associated with a subarachnoid haemorrhage or neural shock, the effects associated with withdrawal from substances of abuse such as cocaine, nicotine, alcohol and benzodiazepines, disorders treatable and/or preventable with anti-convulsive agents, such as epilepsy including post-traumatic epilepsy, Parkinson's disease, psychosis, migraine, cerebral ischaemia, Alzheimer's disease and other degenerative diseases such as Huntingdon's chorea, schizophrenia, obsessive compulsive disorders (OCD), neurological deficits associated with AIDS, sleep disorders (including circadian rhythm disorders, insomnia & narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysthesias in diseases such as diabetes, multiple sclerosis (MS) and motor neurone disease, ataxias, muscular rigidity (spasticity), temporomandibular joint dysfunction and/or amyotrophic lateral sclerosis (ALS). The administration of such compounds in particulate form to a mammal may be by way of oral, parenteral, sub-lingual, nasal, pulmonary, rectal or transdermal administration.

An amount effective to treat the disorders hereinbefore described depends on the usual factors such as the nature and severity of the disorders being treated and the weight of the mammal. However, a unit dose will normally contain 1 to 1000 mg, suitably 1 to 500 mg, for example an amount in the range of from 2 to 400 mg such as 2, 5, 10, 20. 30, 40, 50, 100, 200, 300 and 400 mg of the active compound. Unit doses will normally be administered once or more than once per day, for example 1, 2, 3, 4, 5 or 6 times a day, more usually 1 to 4 times a day, such that the total daily dose is normally in the range, for a 70 kg adult of 1 to 1000 mg, for example 5 to 500 mg, that is in the range of approximately 0.01 to 15 mg/kg/day, more usually 0.1 to 6 mg/kg/day, for example I to 6 mg/kg/day.

It is greatly preferred that the compound of Formula I is administered in the form of a unit-dose composition, such as a unit dose oral, including sub-lingual, nasal. pulmonary, rectal, topical or parenteral (especially intravenous) composition.

Such compositions are prepared by admixture and are suitably adapted for oral, parenteral or pulmonary administration, and as such may be in the form of tablets, capsules, oral liquid preparations, powders, granules, lozenges, reconstitutable powders, injectable and infusable solutions or suspensions or respirable powders or powder suspensions or suppositories. Suitable apparatus for use in administration via the pulmonary route are described for example in US Patents Nos 5,363,842, 5,392,768, 5,394,866, 5,404,871, 5,497,763, 5,509,404, 5,544,646, and 5,608,647. Orally administrable compositions are preferred, in particular shaped oral compositions, since they are more convenient for general use.

Tablets and capsules for oral administration are usually presented in a unit dose, and contain conventional excipients such as binding agents, fillers, diluents, tabletting agents (compression aids), lubricants, disintegrants, colorants, flavourings, and wetting agents. The tablets may be coated according to well known methods in the art.

Suitable fillers for use include cellulose, mannitol, lactose and other similar agents. Suitable disintegrants include starch, cross linked polyvinylpyrrolidone and other super disintegrants and starch derivatives such as sodium starch glycollate. Suitable lubricants include, for example, magnesium stearate.

These solid oral compositions may be prepared by conventional methods of blending, filling, tabletting or the like. Repeated blending operations may be used to distribute the active agent throughout those compositions employing large quantities of fillers. Such operations are, of course, conventional in the art.

Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethyl cellulose. aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol. or ethyl alcohol; preservatives, for example methyl or propyl *p*-hydroxybenzoate or sorbic acid, and if desired conventional flavouring or colouring agents.

Oral formulations also include conventional controlled release formulations, such as tablets or granules having an enteric coating or otherwise modified to control the release of the active compound for example by the inclusion of gel forming polymers such as Methocel K4M.

For parenteral administration, fluid unit dose forms are prepared containing the compound and a sterile vehicle. Parenteral suspensions are normally prepared by suspending the compound in a sterile vehicle, having first sterilized the compound by exposure to ethylene oxide, gamma irradiation or other sterilising process before suspending in the sterile vehicle. Alternatively, the entire process from the recrystallisation of the active compound, the solution of which has previously been sterile filtered, for example through a 0.22 micron membrane filter, can be conducted under aseptic conditions to ensure sterility. Advantageously, a surfactant or wetting agent is included in the composition to facilitate uniform distribution of the compound of the invention.

In a further aspect the invention provides the use of a composition of this invention for the manufacture of a medicament for the treatment and/or prophylaxis of anxiety. mania, depression, panic disorders and/or aggression, disorders associated with a subarachnoid haemorrhage or neural shock, the effects associated with withdrawal from substances of abuse such as cocaine, nicotine, alcohol and benzodiazepines, disorders treatable and/or preventable with anti-convulsive agents, such as epilepsy including post-traumatic epilepsy, Parkinson's disease, psychosis, migraine, cerebral ischaemia, Alzheimer's disease and other degenerative diseases such as Huntingdon's chorea, schizophrenia, obsessive compulsive disorders (OCD), neurological deficits associated with AIDS, sleep disorders (including circadian rhythm disorders, insomnia & narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysthesias in diseases such as diabetes, multiple sclerosis (MS) and motor neurone disease, ataxias, muscular rigidity (spasticity), temporomandibular joint dysfunction and/or amyotrophic lateral sclerosis (ALS).
A preferred procedure for making a small scale particulate composition of this invention can be carried out using a two chamber bead mill,such as a laboratory scale machine, sold under the trade name DENA Mill by Dena Systems BK Ltd, Mapplewell, Barnsley, S75 6DT, with a milling capacity of 2 litres. The mill comprises two reaction chambers SPS 1, SPS 2, and three different reactors D3, S 1 SS. The mill can be operated in three different modes, "process one", "process two" and "process one and two combined". Process one uses the SPS 1 reaction chamber, which contains 1.25 mm beads. Process two uses the SPS 2 reaction chamber, which contains 0.4 mm beads. When the mill is run using process one and two combined the suspension of product to be milled passes through both reaction chambers.

The reaction chambers SPS 1 and SPS2 contain rotating paddles and both the reaction chambers and paddles are manufactured from hard wearing, inert nylon. The inside of the discharge end of the reaction chambers are covered by a sieve of suitable size to stop the beads and any unmilled material leaving the chamber. The rotation speed of the paddles is constant.

The reactors D3, S1 and SS carry out different processes. The reactor D3 is a de-agglomerating reactor; it is designed mainly to de-agglomerate the substance being milled prior to entering the SPS 1 reaction chamber. The reactor S1 is a shearing and emulsifying reactor; it ensures a uniform suspension is produced at the end of the milling process. The SS reactor is designed to homogenise materials and emulsify where appropriate the suspension prior to exiting the mill. Suitable reactors for mixing and homogenizing solid suspensions are described in UK Patent No 2 268 090 B.

The interior of each reactor is a honeycomb baffle structure, arranged to produce angles of flow within the reactor dictated by the function of the reactor. The mill also has two heat exchangers; one for cooling the output of each reaction chamber.

When using this mill in the procedures of the present invention, the mill was operated in accordance with the manufacturer's instructions for "process one and process two combined". so that the compound to be milled was circulated between the two reaction chambers.

The mill chambers were loaded with YTZ® ceramic beads manufactured from yttria-stabilized, high purity zirconia powder (manufactured by Nikkato Corporation. Japan) as follows.

| **Chamber** | **Beads** | |
|---|---|---|
| SPS 1 | Diameter | 1.25mm⁺/- 0.15 mm |
| | Density | 6.07 g/cm³ |
| | Hardness | 12.6Gpa. |
| | Weight | 830 g |
| SPS 2 | Diameter | 0.4 mm + 0.1mm / -0.05mm |
| | Density | 6.07 g/cm³ |
| | Hardness | 12.5Gpa. |
| | Weight | 830 g |

The milled suspension was spray dried using a Mobile Minor Spray Dryer, manufactured by Niro Ltd, Denmark. In this apparatus, the aqueous suspension from the mill is fed by pump into a drying chamber as an atomised spray. The dried powder passes into a cyclone separator from which powdered product is removed.

The Mobile Minor was operated in accordance with the manufacturers instructions at the following settings.

| | | |
|---|---|---|
| Machine Settings | Atomiser Speed | about 25,000 rpm |
| | Exhaust Fan/Heater | Setting II |
| | Pump Speed | 40 rpm |
| | Inlet Temperature | 140°C |
| | Outlet Temperature | 70°C |

Suspensions for milling using the apparatus described above were prepared from:

| | |
|---|---|
| Compound 1 | SB Pharmaceuticals (in unmilled form, particle size typically 1 mm) |
| Compound 2 | As above |
| Mannitol | from Roquette UK Ltd |
| Hydroxypropyl Methylcellulose | from Stancourt Sons and Muir |
| Sodium Lauryl Sulphate | from Henkel Organics |

The proportions of the compounds were varied as detailed below.

Suspensions were prepared by dispersing hydroxypropylmethylcellulose (HPMC) in purified water, using a suitable mixer. The mannitol was added to the hydroxypropyl methylcellulose dispersion, followed by the sodium lauryl sulphate. Then, while mixing at a high speed, the drug under test was dispersed and mixing continued until no drug agglomerates remained.

The following formulations of suspension were investigated.

| **Component** | **Specification** | **Proportion % w/w** | | | | | |
|---|---|---|---|---|---|---|---|
| | **Formulation Number >** | **1** | **2** | **3** | **4** | **5** | **6** |
| Compound 1 | SB Pharmaceuticals | 20 | 15 | 10 | 20 | 30 | 40 |
| | Specification | | | | | | |
| Mannitol | Ph Eur | 10 | 7.5 | 5 | 5 | 5 | 5 |
| HPMC | Ph Eur | 1.5 | 1.5 | 1.5 | 1.5 | 1.5 | 1.5 |
| Sodium | Ph Eur | 0.2 | 0.2 | 0.1 | 0.2 | 0.3 | 0.4 |
| Lauryl Sulphate | | | | | | | |
| Purified Water to | Ph Eur | 100 | to 100 | to 100 | to 100 | to 100 | to 100 |

After milling the suspensions of Formulations 1 - 6, the results are reported as D(0.1), D(0.5), and D(0.9). D(0.1) represents the size (as volume diameter) below which 10% of the particles lie, D(0.5) is the size below which 50% of the particles lie. also known as the median. D(0.9) is the measurement below which 90% of the particles lie.

The particle size distribution was measured using a Malvern Mastersizer S laser diffraction unit from Malvern Instruments Ltd., Malvern. England.

### Suspension

Suspensions containing 10% w/w Compound 1, 15% w/w Compound 1, 20% w/w Compound 1 (Formulations 3,2, 1) were milled successfully, reaching suitable product parameters within 60 minutes (see Tables 1,2, 3). It was found that a 30% w/w suspension (Formulation 5) could be manufactured as a slurry but the bead mill could only process the slurry for a few minutes before the second reaction chamber, SPS 2 (0.4mm beads), became blocked. A 40% w/w slurry (Formulation 6) could not be milled, as it would not suspend long enough for transfer to the mill reservoir.

**Table 1**

| **Time (Minutes)** | **Formulation 1** | | |
|---|---|---|---|
| | D(0.1)µm | D(0.5)µm | D(0.9)µm |
| 0 | ND | ND | ND (Unmicronised Drug) |
| 60 | 0.24 | 0.40 | 0.71 |
| 120 | 0.27 | 0.39 | 0.56 |
| ND - Not determined | | | |

**Table 2**

| **Time (Minutes)** | **Formulation 2** | | |
|---|---|---|---|
| | D(0.1) µm | D(0.5) µm | D(0.9) µm |
| 0 | 0.53 | 10.21 | 73.06 (Micronised Drug) |
| 60 | 0.25 | 0.40 | 0.68 |
| 120 | 0.27 | 0.40 | 0.57 |

**Table 3**

| **Time (Minutes)** | **Formulation 3** | | |
|---|---|---|---|
| | D(0.1) µm | D(0.5) µm | D(0.9) µm |
| 0 | 35.59 | 112.12 | 417.34 (Unmicronised) |
| 60 | 0.25 | 0.41 | 0.77 |
| 120 | 0.25 | 0.39 | 0.60 |

Milling of Formulation 1 was repeated with testing at shorter intervals, as shown in Table 4. Comparison with Table 1 shows that the desirable parameters are reached between 45 and 60 minutes of milling.

**Table 4**

| **Time (Minutes)** | **Formulation 1** | | |
|---|---|---|---|
| | D(0.1) µm | D(0.5) µm | D(0.9) µm |
| 15 | 0.33 | 1.40 | 7.38 |
| 25 | 0.28 | 0.68 | 2.52 |
| 35 | 0.28 | 0.62 | 1.99 |
| 45 | 0.27 | 0.60 | 1.93 |

The suspension of Formulation 4 was milled for 70 minutes and samples analysed for particle size at 10 minute intervals (Table 5). It can be seen that a desirable particle size was achieved in 30 minutes milling time. After 30 minutes milling no more significant particle size reduction was achieved.

**Table 5**

| **Time (Minutes)** | **Formulation 4** | | |
|---|---|---|---|
| | D(0.1) µm | D(0.5) µm | D(0.9) µm |
| 0 | 27.27 | 85.96 | 343.72 (Unmicronised) |
| 10 | 0.27 | 0.58 | 1.66 |
| 20 | 0.27 | 0.54 | 1.54 |
| 30 | 0.26 | 0.53 | 1.45 |
| 50 | 0.26 | 0.48 | 1.26 |
| 60 | 0.26 | 0.45 | 0.95 |
| 70 | 0.25 | 0.44 | 0.93 |

For all suspensions with a mannitol level of 5% to 10% spray drying was successful, as was the redispersion of the spray dried material.

To demonstrate that the process is reproducible, six batches (A - F) of 20kg of the suspension of Formulation 4 were milled in sub-lots of 2kg. Sixty lots were milled, and particle size analysis was carried out on a sample of the 20kg bulk. For all the six 20kg lots the desirable particle size was achieved (Table 6) after 30 minutes milling time.
The standard deviation for the three measurement areas is very low, showing no batch to batch variation.
(D(0.1) 4 x 10⁻³, D(0.5) 5.5 x 10⁻³, D(0.9) 6 x 10⁻³.

**Table 6**

| **Formulation** | **D(0.1) µm** | **D(0.5) µm** | **D(0.9) µm** |
|---|---|---|---|
| 4 A | 0.27 | 0.49 | 1.03 |
| 4 B | 0.26 | 0.49 | 1.16 |
| 4 C | 0.26 | 0.50 | 1.23 |
| 4 D | 0.26 | 0.50 | 1.18 |
| 4 E | 0.26 | 0.50 | 1.21 |
| 4 F | 0.26 | 0.49 | 1.16 |

A preferred procedure for making a commercial scale particulate composition of this invention may be carried out using a five chamber bead mill such as a commercial scale machine, sold under the trade name DENA DS-1P5 Mill by Dena Systems BK Ltd, Mapplewell, Barnsley, S75 6DT, with a milling capacity of 2 to 3 litres per minute. The mill comprises five reaction chambers SPS 1 to SPS 5, and seven different reactors (type D, S and SS). The mill is operated in one mode by passing product once through each of the mill chambers. Depending on the product, between one and five mill chambers can be used.

The reaction chambers SPS 1 to SPS5 and the reactors are of similar design to the small scale mill referred to above

The mill chambers were loaded with YTZ® ceramic beads manufactured from yttria-stabilized, high purity zirconia powder (manufactured by Nikkato Corporation, Japan) as follows. Bead sizes range between 0.3mm and 1.25mm in diameter. Bead loadings in each chamber vary from 6250 cc to 6750cc

The milled suspension was spray dried using a Niro Spray Dryer. manufactured by Niro Ltd, Denmark. In this apparatus, the aqueous suspension from the mill is fed by pump into a drying chamber as an atomised spray. The dried powder passes into a cyclone separator from which powdered product is removed.

The spray drier was operated in accordance with the manufacturers instructions at the following settings.

| | | |
|---|---|---|
| Machine Settings | Atomiser Speed | 21,000 to about 25,000 rpm |
| | Pump Speed | rpm |
| | Inlet Temperature | 140°C |
| | Outlet Temperature | 70°C |

Suspensions for milling using the apparatus described above were prepared from identical excipients and active as outlined above for the small scale milling experiments:

The following formulations of suspension were investigated.

| **Component** | **Specification** | **Proportion % w/w** |
|---|---|---|
| | **Formulation Number >** | **1** |
| Compound 1 | SB Pharmaceuticals | 20 |
| | Specification | |
| Mannitol | Ph Eur | 10 |
| HPMC | Ph Eur | 1.5 |
| Sodium | Ph Eur | 0.2 |
| Lauryl Sulphate | | |
| Purified Water | Ph Eur | to 100 |

After milling the suspensions of Formulation 1 using different mill chamber bead loadings and alternative bead sizes, the range of results are reported in Table 7 as D(0.1), D(0.5), and D(0.9). D(0.1) represents the size (as volume diameter) below which 10% of the particles lie, D(0.5) is the size below which 50% of the particles lie, also known as the median. D(0.9) is the measurement below which 90% of the particles lie.

The particle size distribution was measured using a Malvern Mastersizer S laser diffraction unit from Malvern Instruments Ltd., Malvern. England.

### Suspension

**Table 7**

| **Mill chamber** | **Formulation 1** | | |
|---|---|---|---|
| | D(0.1)µm | D(0.5) µm | D(0.9) µm |
| 0 | 36 | 112 | 417 (Unmicronised Drug) |
| 1 | 0.25 to 0.46 | 2.4 to 4.2 | 10.3 to 18 |
| 2 | 0.32 to 0.34 | 1.1 to 1.3 | 5.0 to 6.5 |
| 3 | 0.29 to 0.30 | 0.71 to 0.87 | 2.8 to 4.5 |
| 4 | 0.25 to 0.30 | 0.54 to 0.81 | 1.4 to 2.1 |
| 5 | 0.25 to 0.28 | 0.48 to 0.52 | 1.0 to 1.3 |

The levels of yttrium and zirconium in all milled suspensions were assayed using inductively coupled plasma analysis and found to be at low and acceptable levels. The iron levels were also low. An acceptable level for zirconium was set at < 200ppm Zr, and yttrium 20ppm in the spray dried powder. The results show that the beads are not breaking down during the milling process and the nylon mill chambers are not being damaged by the beads or the suspension.

### Bio-Equivalence Study

A bio-equivalence study to determine the relative bioavailability of four oral formulations of Compound 1 in healthy volunteers was perfomed.
The study design comprised an open, randomised, four part crossover. Each subject was to receive, randomly. on separate days, a single oral 400 mg dose of each of the four different formulations of Compound 1.Compound 1 was administered orally in tablet or capsule form. On each of the four dosing days subjects were to receive a single oral dose of Compound 1, with a washout of at least 6 days between doses. For each subject the four formulations were randomly allocated to each study session. Doses were administered orally with 200 mL water, following a standard breakfast. The spray dried product from the suspension of Formulation 4 was compressed into tablets by direct compression.

A control tablet formulation was as follows:

| **Excipient** | **mg/tablet** |
|---|---|
| Compound 1 (micronised) | 400.00 |
| Sodium starch glycollate | 24.00 |
| Microcrystalline cellulose Ph.Eur. | 23.28 |
| Polyvinylpyrrolidone USP | 9.60 |
| Sodium lauryl sulphate BP | 2.40 |
| Lactose (monohydrate) Ph.Eur. | 18.48 |
| Magnesium stearate Ph.Eur. | 2.40 |
| Purified water Ph.Eur. | q.s. (removed during processing) |
| Total tablet weight | 480.16 |

The test conditions were the same for both formulations. The results (Tables 10 and 11) show an increase in blood levels of Compound 1 by approximately 3-fold when dosing with tablets of Formulation 4 when compared to the tablet sourced from micronised drug substance.

**Table 10**

| | **Control Tablet** | **Wet Milled Spray Dried Tablet** |
|---|---|---|
| AUC (mg.hr/ml) | 11.8 | 31.5 |
| C MAX. (mg/ml) | 1.07 | 3.03 |
| tmax/hr | 3.8 | 3.3 |
| AUC - Area under the curve C MAX - Maximum plasma concentration tmax - Time to maximum plasma level | | |

**Table 11**

| **Results for Bio-equivalence study** | | |
|---|---|---|
| **Time** | **Control Tablet** | **Wet Milled Spray Dried Tablet** |
| **(Hours)** | **(plasma concentration µg/ml)** | |
| 0 | 0.00 | 0.00 |
| 0.48 | 0.20 | 0.20 |
| 1.0 | 0.35 | 0.65 |
| 1.5 | 0.50 | 1.30 |
| 2.0 | 0.70 | 2.40 |
| 3.0 | 1.00 | 2.75 |
| 4.0 | 0.95 | 3.00 |
| 6.0 | 0.90 | 2.30 |
| 8.0 | 0.69 | 1.75 |
| 10.0 | 0.65 | 1.50 |
| 12.0 | 0.60 | 1.30 |
| 24.0 | 0.50 | 0.80 |

### Rat maximal electroshock seizure threshold (MEST) test

For full details see Upton *et al* (1997, *Br.J. Pharmacol*., **121,** 1679-1686)

The threshold for electroshock-induced tonic hindlimb extensor seizures in male rats (Sprague Dawley strain, 70-130g) was determined using a Hugo Sachs Elektronic stimulator which delivered a constant current (0.3 s duration. 50 Hz, sinewave form, fully adjustable between 1-300 mA) via corneal electrodes. The stimulus intensity was varied (from the typical baseline current of 25 mA) by an "up-and-down" method of shock titration using current steps of 5-20 mA. Hindlimb extension was scored as absent or present. The data generated from treatment groups of 10-14 rats were used to calculate the CC₅₀ value (current producing maximal seizures of 50% of animals) ± s.e.m. according to the method of Kimball *et al* (1957, *Radiation Res.* **7**, 1-12). The effects of the various drug treatments were expressed as the % change in CC₅₀ values compared to vehicle-treated control levels. An increase in seizure threshold is indicative of an anticonvulsant effect. Significant effects (*P* < 0.05) of drugs on the threshold for maximal electroshock seizures were determined by comparison of the potency ratios of the CC₅₀ values of the drug and vehicle-treated groups by the method of Litchfield and Wilcoxan (1949, *J. Pharmacol. Exp. Ther*. **96,** 99-113).

Test drugs were administered orally by gavage using a dose volume of 1 ml/kg.

### Anticonvulsant Effect of Different Formulations of Compound 2 (5 mg/kg p.o.) in the Rat MEST Test

| **Pre-Test Time** | | **% Increase in Seizure Threshold** | | | |
|---|---|---|---|---|---|
| **(mins)** | **1% methyl cellulose** | **micronised** ^{**a**} | **wet-milled** ^{**b**} **+ SLS** | **wet -milled** ^{**c**} | **formulation 4** ^{**d**} |
| 15 | 76 | 114 | 181 | 182 | 184 |
| 30 | 202 | 231 | 369 | 335 | 410 |
| 60 | 194 | 369 | 444 | 424 | 492 |
| 90 | - | 549 | 557 | 410 | 580 |
| 120 | 576 | 798 | 526 | 839 | 893 |
| 15 min/2hr | 11% | 14% | 34% | 22% | 21% |
| Conclusions: 1. **c** and **d** have better overall effect than **a** 2. **b**, **c** & **d** have faster rate of onset than **a** | | | | | |

## Claims

1. A pharmaceutical composition comprising a compound of Formula I or Formula II below in particulate form, said composition having a monomodal particle size distribution such that the median value of the volume mean diameter is within the range of from 350 to 700 nm, typically with no more than 10% of the particles having a volume diameter of 280 nm or below, and no less than 90% of the particles having a volume diameter of 2000 nm or below: 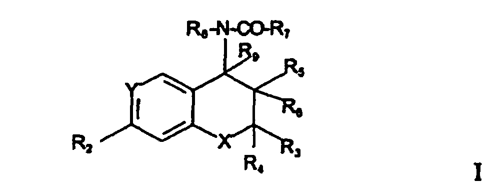 wherein:
either Y is N and R₂ is hydrogen, or Y is C-R₁
where:
either one of R₁ and R₂ is hydrogen and the other is selected from the class of hydrogen, C₃₋₈ cycloalkyl, C₁₋₆ alkyl optionally interupted by oxygen or substituted by hydroxy, C₁₋₆ alkoxy or substituted aminocarbonyl, C₁₋₆ alkylcarbonyl, C₁₋₆ alkoxycarbonyl, C₁₋₆ alkylcarbonyloxy, C₁₋₆ alkoxy, nitro, cyano, halo, trifluoromethyl, CF₃S, or a group CF₃-A-, where A is -CF₂-, -CO-, -(CH₂- or CH(OH), trifluoromethoxy, C₁₋₆ alkylsulphinyl, C₁₋₆ alkylsulphonyl, C₁₋₆ alkoxysulphinyl, C₁₋₆ alkoxysulphonyl, aryl, heteroaryl, arylcarbonyl, heteroarylcarbonyl, arylsulphinyl, heteroarylsulphinyl, arylsulphonyl, heteroarylsulphonyl in which any aromatic moiety is optionally substituted, C₁₋₆ alkylcarbonylamino, C₁₋₆ alkoxycarbonylamino, C₁₋₆ alkyl-thiocarbonyl, C₁₋₆ alkoxy-thiocarbonyl, C₁₋₆ alkyl-thiocarbonyloxy, 1-mercapto C₂₋₇ alkyl, formyl, or aminosulphinyl, aminosulphonyl or aminocarbonyl, any amino moiety being optionally substituted by one or two C₁₋₆ alkyl groups, or C₁₋₆ alkylsulphinylamino, C₁₋₆ alkylsulphonylamino,C₁₋₆ alkoxysulphinylamino or C₁₋₆ alkoxysulphonylamino, or ethylenyl terminally substituted by C₁₋₆ alkylcarbonyl, nitro or cyano, or -C(C₁₋₆ alkyl)NOH or -C(C₁₋₆ alkyl)NNH₂, or one of R₁ and R₂ is nitro, cyano or C₁₋₃ alkylcarbonyl and the other is methoxy or amino optionally substituted by one or two C₁₋₆ alkyl or by C₂₋₇ alkanoyl;
one of R₃ and R₄ is hydrogen or C₁₋₄ alkyl and the other is C₁₋₄ alkyl or R₃ and R₄ together are C₂₋₅ polymethylene;
R₅ is C₁₋₆ alkylcarbonyloxy, benzoyloxy, ONO₂, benzyloxy, phenyloxy or C₁₋₆ alkoxy and R₆ and R₉ are hydrogen or R₅ is hydroxy and R₆ is hydrogen or C₁₋₂ alkyl and R₉ is hydrogen;
R₇ is fluorophenyl;
R₈ is hydrogen or C₁₋₆ alkyl;
the R₈-N-CO-R₇ group being *trans* to the R₅ group;
and X is oxygen or NR₁₀ where R₁₀ is hydrogen or C₁₋₆ alkyl;
and 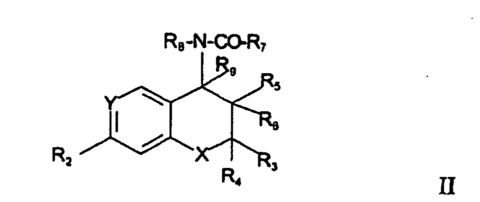 wherein:
either Y is N and R₂ is hydrogen, or Y is C-R₁
where:
either one of R₁ and R₂ is hydrogen and the other is selected from the class of hydrogen, C₃₋₈ cycloalkyl, C₁₋₆ alkyl optionally interrupted by oxygen or substituted by hydroxy, C₁₋₆ alkoxy or substituted aminocarbonyl, C₁₋₆ alkylcarbonyl, C₁₋₆ alkoxycarbonyl, C₁₋₆ alkylcarbonyloxy, C₁₋₆ alkoxy, nitro, cyano, halo, trifluoromethyl, CF₃S, or a group CF₃-A-, where A is -CF₂-, -CO-, -CH₂-, CH(OH), SO₂, SO, CH₂-O, or CONH, or a group CF₂H-A'- where A' is oxygen, sulphur, SO, SO₂, CF₂ or CFH; trifluoromethoxy, C₁₋₆ alkylsulphinyl, perfluoro C₂₋₆ alkylsulphonyl, C₁₋₆ alkylsulphonyl, C₁₋₆ alkoxysulphinyl, C₁₋₆ alkoxysulphonyl, aryl, heteroaryl, arylcarbonyl, heteroarylcarbonyl, phosphono, arylcarbonyloxy, heteroarylcarbonyloxy, arylsulphinyl, heteroarylsulphinyl, arylsulphonyl, heteroarylsulphonyl in which any aromatic moiety is optionally substituted, C₁₋₆ alkylcarbonylamino, C₁₋₆ alkoxycarbonylamino, C₁₋₆ alkyl-thiocarbonyl, C₁₋₆ alkoxy-thiocarbonyl, C₁₋₆ alkyl-thiocarbonyloxy, 1-mercapto C₂₋₇ alkyl, formyl, or aminosulphinyl, aminosulphonyl or aminocarbonyl, any amino moiety being optionally substituted by one or two C₁₋₆ alkyl groups, or C₁₋₆ alkylsulphinylamino, C₁₋₆ alkylsulphonylamino,C₁₋₆ alkoxysulphinylamino or C₁₋₆ alkoxysulphonylamino, or ethylenyl terminally substituted by C₁₋₆ alkylcarbonyl, nitro or cyano, or -C(C₁₋₆ alkyl)NOH or -C(C₁₋₆ alkyl)NNH₂, or one of R₁ and R₂ is nitro, cyano or C₁₋₃ alkylcarbonyl and the other is methoxy or amino optionally substituted by one or two C₁₋₆ alkyl or by C₂₋₇ alkanoyl; or R₁ and R₂ together are -(CH₂)₄- or -CH = CH-CH = CH-, or form an optionally substituted triazole or oxadiazole ring;
one of R₃ and R₄ is hydrogen or C₁₋₄ alkyl and the other is C₁₋₄ alkyl, CF₃ or CH₂ X^{a} where X^{a} is fluoro, chloro, bromo, iodo, C₁₋₄ alkoxy, hydroxy, C₁₋₄ alkylcarbonyloxy, -S-C₁₋₄ alkyl, nitro, amino optionally substituted by one or two C₁₋₄ alkyl groups; cyano or C₁₋₄ alkoxycarbonyl or R₃ and R₄ together are C₂₋₅ polymethylene optionally substituted by C₁₋₄ alkyl;
R₅ is C₁₋₆ alkylcarbonyloxy, benzoyloxy, ONO₂, benzyloxy, phenyloxy or C₁₋₆ alkoxy and R₆ and R₉ are hydrogen or R₅ is hydroxy and R₆ is hydrogen or C₁₋₂ alkyl and R₉ is hydrogen;
R₇ is heteroaryl or phenyl; both of which are optionally substituted one or more times independently with a group or atom selected from chloro, fluoro, bromo, iodo, nitro, amino optionally substituted once or twice by C₁₋₄ alkyl, cyano, azido, C₁₋₄ alkyl, C₁₋₄ alkoxy, trifluoromethoxy and trifluoromethyl;
R₈ is hydrogen; C₁₋₆ alkyl, OR₉ or NHCOR₁₀ wherein R₉ is hydrogen, C₁₋₆ alkyl, formyl, C₁₋₆ alkanoyl, aroyl or aryl-C₁₋₆ alkyl and R₁₀ is hydrogen, C₁₋₆ alkyl, C₁₋₆ alkoxy, mono or di C₁₋₆ alkyl amino, amino, amino-C₁₋₆ alkyl, hydroxy-C₁₋₆ alkyl, halo-C₁₋₆ alkyl, C₁₋₆ acyloxy-C₁₋₆ alkyl, C₁₋₆ alkoxycarbonyl-C₁₋₆-alkyl, aryl or heteroaryl;
the R₈-N-CO-R₇ group being *cis* to the R₅ group;
and X is oxygen or NR₁₀ where R₁₀ is hydrogen or C₁₋₆ alkyl.

2. A composition according to any claims 1 in which the median volume diameter is in the range of 450 to 550 nm.

3. A composition according to claim 2 in which 10% of particles have a volume diameter of 260 nm or below and 90% of particles have a volume diameter of 1450 nm or below.

4. A composition according to any one of claims 1 to 3 which is obtainable by wet milling compounds of Formula I and Formula II as an aqueous dispersion.

5. A composition according to claim 4 which is obtainable by wet milling using a multi-chamber bead mill with single pass or recirculation of product between the chambers.

6. A composition according to claim 4 or 5 which is obtainable using ceramic beads of rare earth oxides as milling medium.

7. A composition according to claim 5 or 6 which is obtainable using a multichamber chamber mill in which the beads in one chamber are of a smaller diameter than the beads in the other chambers.

8. A composition according to claim 7 which is obtainable by using a range of bead sizes between 0.4mm and 1.25mm in each chamber

9. A composition according to any one of claims 1 to 8 including a soluble carrier suitable as an excipient for spray drying.

10. A composition according to claim 8 in which the soluble carrier is mannitol.

11. A composition according to any one of claims 1 to 10 including a surfactant to maintain the particles in suspension during milling and on re-suspension.

12. A composition according to claim 11 in which the surfactant is sodium lauryl sulphate.

13. A composition according to any one of claims 1 to 12 including an anti-agglomeration agent effective after administration of a pharmaceutical formulation to a patient.

14. A composition according to claim 13 in which the anti-agglomeration agent is hydroxypropyl methyl cellulose.

15. A process for preparing a particulate composition in which the drug substance is a compound of formula I or II according to claim 1 which comprises:
a) wet milling the drug substance as an aqueous dispersion in a multi-chamber bead mill with circulation of product between the chambers, the aqueous dispersion including a soluble carrier suitable as an excipient for spray drying, a surfactant to maintain the particles in suspension on re-suspension, and an anti-agglomeration agent effective after administration of a pharmaceutical formulation to a patient; and
b) spray drying the aqueous dispersion after milling.

16. A process according to claim 15 in which the aqueous dispersion to be milled comprises about 10 to 30% w/w of a compound of Formula I or II, about 4 to 15% w/w of the soluble carrier, about 0.1 to 0.4% w/w of surfactant, and about 1% w/w to 2% w/w of the anti-agglomeration agent.

17. The use of a composition according to any one of claims 1 to 14 for the manufacture of a medicament for the treatment and/or prophylaxis of anxiety, mania, depression, panic disorders and/or aggression, disorders associated with a subarachnoid haemorrhage or neural shock, the effects associated with withdrawal from substances of abuse such as cocaine, nicotine, alcohol and benzodiazepines, disorders treatable and/or preventable with anti-convulsive agents, such as epilepsy including post-traumatic epilepsy, Parkinson's disease, psychosis, migraine, cerebral ischaemia, Alzheimer's disease and other degenerative diseases such as Huntingdon's chorea, schizophrenia, obsessive compulsive disorders (OCD), neurological deficits associated with AIDS, sleep disorders (including circadian rhythm disorders, insomnia & narcolepsy), tics (e.g. Giles de la Tourette's syndrome), traumatic brain injury, tinnitus, neuralgia, especially trigeminal neuralgia, neuropathic pain, dental pain, cancer pain, inappropriate neuronal activity resulting in neurodysthesias in diseases such as diabetes, multiple sclerosis (MS) and motor neurone disease, ataxias, muscular rigidity (spasticity), temporomandibular joint dysfunction and/or amyotrophic lateral sclerosis (ALS).

## Patentansprüche

1. Pharmazeutische Zusammensetzung, welche eines der nachstehenden Komponenten der Formel I oder der Formel II in Teilchenform umfasst, wobei die Zusammensetzung eine monomodale Teilchengrößenverteilung besitzt, so dass der Median des Volumenmittels des Durchmessers innerhalb des Bereiches von 350 bis 700 nm liegt, wobei typischerweise nicht mehr als 10 % der Partikel einen Volumendurchmesser von 280 nm oder weniger aufweisen und nicht mehr als 90 % der Partikel einen Volumendurchmesser von 2000 nm oder weniger aufweisen: 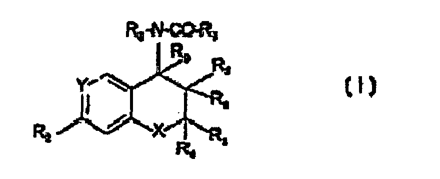 wobei:
entweder Y N ist und R₂ Wasserstoff ist oder Y C-R₁ ist;
wobei:
einer der Reste R₁ und R₂ Wasserstoff ist und der andere ausgewählt ist aus Wasserstoff, C₃₋₈-Cycloalkyl, C₁₋₆-Alkyl gegebenenfalls unterbrochen von Sauerstoff oder substituiert mit Hydroxy, C₁₋₆-Alkoxy oder substituiertem Aminocarbonyl, C₁₋₆-Alkylcarbonyl, C₁₋₆-Alkoxycarbonyl, C₁₋₆-Alkylcarbonyloxy, C₁₋₆-Alkoxy, Nitro, Cyano, Halogen, Trifluormethyl, CF₃S oder einem Rest CF₃-A-, wobei A -CF₂-, -CO-, -CH₂- oder CH(OH) ist, Trifluormethoxy, C₁₋₆-Alkylsulfinyl, C₁₋₆-Alkylsulfonyl, C₁₋₆-Alkoxysulfinyl, C₁₋₆-Alkoxysulfonyl, Aryl, Heteroaryl, Arylcarbonyl, Heteroarylcarbonyl, Arylsulfinyl, Heteroarylsulfinyl, Arylsulfonyl, Heteroarylsulfonyl, wobei jede aromatische Einheit gegebenenfalls substituiert ist, C₁₋₆-Alkylcarbonylamino, C₁₋₆-Alkoxycarbonylamino, C₁₋₆-Alkylthiocarbonyl, C₁₋₆-Alkoxythiocarbonyl, C₁₋₆-Alkylthiocarbonyloxy, 1-Mercapto-C₂₋₇-alkyl, Formyl oder Aminosulfinyl, Aminosulfonyl oder Aminocarbonyl, wobei jede Aminoeinheit gegebenenfalls substituiert ist mit ein oder zwei C₁₋₆-Alkylresten oder C₁₋₆-Alkylsulfinylamino, C₁₋₆-Alkylsulfonylamino, C₁₋₆-Alkoxysulfinylamino oder C₁₋₆-Alkoxysulfonylamino oder Ethylenyl, welches endständig substituiert ist mit einem C₁₋₆-Alkylcarbonyl, Nitro oder Cyano oder -C(C₁₋₆-Alkyl)NOH oder -C(C₁₋₆-Alkyl)NNH₂ oder einer der Reste R₁ und R₂ ein Nitro, Cyano oder C₁₋₃-Alkylcarbonyl ist und der andere Methoxy oder Amino ist, das gegebenenfalls mit einem oder zwei C₁₋₆-Alkyl oder mit C₂₋₇-Alkanoyl substituiert ist;
einer der Reste R₃ und R₄ Wasserstoff oder C₁₋₄-Alkyl ist und der andere C₁₋₄-Alkyl ist oder R₃ und R₄ zusammen C₂₋₅-Polymethylen sind;
R₅ C₁₋₆-Alkylcarbonyloxy, Benzoyloxy; ONO₂, Benzyloxy, Phenyloxy oder C₁₋₆-Alkoxy ist und R₆ und R₉ Wasserstoff sind oder R₅ Hydroxy ist und R₆ Wasserstoff oder C₁₋₂-Alkyl ist und R₉ Wasserstoff ist;
R₇ Fluorophenyl ist;
R₈ Wasserstoff oder C₁₋₆-Alkyl ist;
wobei der Rest R₈-N-CO-R₇ *trans* zum R₅ Rest ist;
und X Sauerstoff oder NR₁₀ ist, wobei R₁₀ Wasserstoff oder C₁₋₆-Alkyl ist;
und 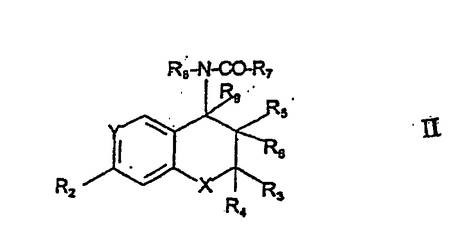 wobei:
entweder Y N ist und R₂ Wasserstoff ist oder Y C-R₁ ist;
wobei:
einer der Reste R₁ und R₂ Wasserstoff ist und der andere ausgewählt ist aus Wasserstoff, C₃₋₈-Cycloalkyl, C₁₋₆-Alkyl gegebenenfalls unterbrochen von Sauerstoff oder substituiert mit Hydroxy, C₁₋₆-Alkoxy oder substituiertem Aminocarbonyl, C₁₋₆-Alkylcarbonyl, C₁₋₆-Alkoxycarbonyl, C₁₋₆-Alkylcarbonyloxy, C₁₋₆-Alkoxy, Nitro, Cyano, Halogen, Trifluormethyl, CF₃S oder einem Rest CF₃-A-, wobei A -CF₂-, -CO-, -CH₂-, CH(OH), SO₂, SO, CH₂-O oder CONH ist, oder einem Rest CF₂H-A'-, wobei A' Sauerstoff, Schwefel, SO, SO₂, CF₂ oder CFH ist; Trifluormethoxy, C₁₋₆-Alkylsulfinyl, Perfluoro-C₂₋₆-alkylsulfonyl, C₁₋₆-Alkylsulfonyl, C₁₋₆-Alkoxysulfinyl, C₁₋₆-Alkoxysulfonyl, Aryl, Heteroaryl, Arylcarbonyl, Heteroarylcarbonyl, Phosphono, Arylcarbonyloxy, Heteroarylcarbonyloxy, Arylsulfinyl, Heteroarylsulfinyl, Arylsulfonyl, Heteroarylsulfonyl, wobei jede aromatische Einheit gegebenenfalls substiuiert ist, C₁₋₆-Alkylcarbonylamino, C₁₋₆-Alkoxycarbonylamino, C₁₋₆-Alkylthiocarbonyl, C₁₋₆-Alkoxythiocarbonyl, C₁₋₆-Alkylthiocarbonyloxy, 1-Mercapto-C₂₋₇-Alkyl, Formyl oder Aminosulfinyl, Aminosulfonyl oder Aminocarbonyl, wobei jede Aminoeinheit gegebenenfalls substituiert ist mit ein oder zwei C₁₋₆-Alkylresten oder C₁₋₆-Alkylsulfinylamino, C₁₋₆-Alkylsulfonylamino, C₁₋₆-Alkoxysulfinylamino oder C₁₋₆-Alkoxysulfonylamino oder Ethylenyl, welches endständig substituiert ist mit C₁₋₆-Alkylcarbonyl, Nitro oder Cyano oder -C(C₁₋₆-Alkyl)NOH oder -C(C₁₋₆-Alkyl)NNH₂ oder einer der Reste R₁ und R₂ ein Nitro, Cyano oder C₁₋₃-Alkylcarbonyl ist und der andere ein Methoxy oder Amino ist, das gegebenenfalls mit einem oder zwei C₁₋₆-Alkyl oder mit C₂₋₇-Alkanoyl substituiert ist; oder R₁ und R₂ zusammen -(CH₂)₄- oder -CH=CH-CH=CH- sind oder einen gegebenenfalls substituierten Triazol- oder Oxadiazolring bilden;
einer der Reste R₃ und R₄ Wasserstoff oder C₁₋₄-Alkyl ist und der andere C₁₋₄-Alkyl, CF₃ oder CH₂X^{a} ist, wobei X^{a} Fluor, Chlor, Brom, Iod, C₁₋₄-Alkoxy, Hydroxy, C₁₋₄-Alkylcarbonyloxy, -S-C₁₋₄-Alkyl, Nitro, Amino, das gegebenenfalls mit einer oder zwei C₁₋₄-Alkylresten substituiert ist; Cyano oder C₁₋₄-Alkoxycarbonyl ist oder R₃ und R₄ zusammen gegebenenfalls mit C₁₋₄-Alkyl substituiertes C₂₋₅-Polymethylen sind;
R₅ C₁₋₆-Alkylcarbonyloxy, Benzoyloxy, ONO₂, Benzyloxy, Phenyloxy oder C₁₋₆-Alkoxy ist und R₆ und R₉ Wasserstoff sind oder R₅ Hydroxy ist und R₆ Wasserstoff oder C₁₋₂-Alkyl ist und R₉ Wasserstoff ist;
R₇ Heteroaryl oder Phenyl ist, wobei beide gegebenenfalls ein oder mehrmals unabhängig mit Chlor, Fluor, Brom, Iod, Nitro, Amino, welches gegebenenfalls ein oder zweimal mit C₁₋₄-Alkyl substituiert ist, Cyano, Azido, C₁₋₄-Alkyl, C₁₋₄-Alkoxy, Trifluormethoxy oder Trifluormethyl substituiert sind;
R₈ Wasserstoff, C₁₋₆-Alkyl, OR₉ oder NHCOR₁₀ ist, wobei R₉ Wasserstoff, C₁₋₆-Alkyl, Formyl, C₁₋₆-Alkanoyl, Aroyl oder Aryl-C₁₋₆-alkyl ist und R₁₀ Wasserstoff, C₁₋₆-Alkyl, C₁₋₆-Alkoxy, mono- oder di-C₁₋₆-Alkylamino, Amino, Amino-C₁₋₆-alkyl, Hydroxy-C₁₋₆-alkyl, Halogen-C₁₋₆-alkyl, C₁₋₆-Acyloxy-C₁₋₆-alkyl, C₁₋₆-Alkoxycarbonyl-C₁₋₆-alkyl, Aryl oder Heteroaryl ist;
wobei der Rest R₈-N-CO-R₇ *cis* zum R₅ Rest ist;
und X Sauerstoff oder NR₁₀ ist, wobei R₁₀ Wasserstoff oder C₁₋₆-Alkyl ist.

2. Zusammensetzung gemäß Anspruch 1, wobei der mittlere Volumendurchmesser im Bereich von 450 bis 550 nm liegt.

3. Zusammensetzung gemäß Anspruch 2, wobei 10% der Teilchen einen Volumendurchmesser von 260 nm oder weniger und 90 % der Teilchen einen Volumendurchmesser von 1450 nm oder weniger aufweisen.

4. Zusammensetzung gemäß einem der Ansprüche 1 bis 3, welche durch feuchtes Mahlen der Komponenten der Formel I und der Formel II als eine wässrige Dispersion erhältlich ist.

5. Zusammensetzung gemäß Anspruch 4, welche durch feuchtes Mahlen unter Verwendung einer Mehrkammerperlenreibmühle mit Einzeldurchgang oder Rezirkulation des Produkts zwischen den Kammern erhältlich ist.

6. Zusammensetzung gemäß Anspruch 4 oder 5, welche mit Keramikperlen seltener Erdoxide als Mahlmedium erhältlich ist.

7. Zusammensetzung gemäß Anspruch 5 oder 6, welche unter Verwendung einer Mehrkammer-Kammermühle erhältlich ist, wobei die Perlen in einer Kammer einen kleineren Durchmesser aufweisen als die Perlen in den anderen Kammern.

8. Zusammensetzung gemäß Anspruch 7, welche unter Verwendung von Perlengrößen in einem Bereich zwischen 0,4 mm und 1,25 mm in jeder Kammer erhältlich ist.

9. Zusammensetzung gemäß einem der Ansprüche 1 bis 8, die eine lösliche Trägersubstanz, welche als Exzipient zur Sprühtrocknung geeignet ist, enthält.

10. Zusammensetzung gemäß Anspruch 8, wobei die lösliche Trägersubstanz Mannitol ist.

11. Zusammensetzung gemäß einem der Ansprüche 1 bis 10, die einen oberflächenaktiven Stoff enthält, um die Teilchen während dem Mahlen und der Resuspension in Suspension zu halten.

12. Zusammensetzung gemäß Anspruch 11, wobei der oberflächenaktive Stoff Natriumlaurylsulfat ist.

13. Zusammensetzung gemäß einem der Ansprüche 1 bis 12, die ein Antiagglomerationsmittel, das nach Verabreichung einer pharmazeutischen Formulierung an einen Patienten wirksam ist, enthält.

14. Zusammensetzung gemäß Anspruch 13, wobei das Antiagglomerationsmittel Hydroxypropylmethylcellulose ist.

15. Verfahren zur Herstellung einer teilchenförmigen Zusammensetzung, wobei die Arzneimittelsubstanz eine Verbindung der Formel I oder II nach Anspruch 1 ist, umfassend:
a) feuchtes Mahlen der Arzneimittelsubstanz als eine wässrige Dispersion in einer Mehrkammerperlenreibmühle mit Zirkulation des Produkts zwischen den Kammern, wobei die wässrige Dispersion eine lösliche Trägersubstanz, welches als Exzipient zur Sprühtrocknung geeignet ist, einen oberflächenaktiven Stoff, um die Teilchen bei der Resuspension in Suspension zu halten, und ein Antiagglomerationsmittel, das nach Verabreichung einer pharmazeutischen Formulierung an einem Patienten wirksam ist, enthält; und
b) Sprühtrocknung der wässrigen Dispersion nach dem Mahlen.

16. Verfahren gemäß Anspruch 15, wobei die wässrige Dispersion, die gemahlen werden soll, etwa 10 bis 30% w/w einer Komponente der Formel I oder II, etwa 4 bis 15% w/w der löslichen Trägersubstanz, etwa 0,1 bis 0,4% w/w des oberflächenaktiven Stoffes und etwa 1% w/w bis 2% w/w des Antiagglomerationsmittels umfasst.

17. Verwendung einer Zusammensetzung nach einem der Ansprüche 1 bis 14 zur Herstellung eines Medikaments zur Behandlung und/oder Prophylaxe von Angst, Manie, Depression, Panikstörungen und/oder Aggression, Störungen verbunden mit einer subarachnoidalen Hämorraghie oder Neuralschock, Effekten verbunden mit dem Entzug von Missbrauchssubstanzen, solche wie Kokain, Nikotin, Alkohol und Benzodiazepine, Störungen behandelbar und/oder vermeidbar mit Antikonvulsiva, wie Epilepsie einschließlich posttraumatische Epilepsie, Parkinsonerkrankung, Psychose, Migräne, zerebrale Ischämie, Alzheimer Erkrankung und anderen degenerativen Erkrankungen wie Chorea Huntington, Schizophrenie, Zwangsstörungen (OCD), neurologischen Störungen verbunden mit AIDS, Schlafstörungen (einschließlich Herzrhythmusstörungen, Schlaflosigkeit & Narkolepsie), Tic (z.B. Gilles-de-la-Tourette-Syndrom), traumatischen Gehirnverletzung, Tinnitus, Neuralgie, insbesondere Trigeminusneuralgie, neuropathischem Schmerz, Zahnschmerz, Krebsschmerz, unangemessenen Neuronenaktivität, die zu Neuraldysthesien bei solchen Krankheiten führen, wie Diabetes, Multiple Sklerose (MS) und Motorneuronenkrankheit, Ataxie, Muskelsteifheit (Spastik), Fehlfunktion des mandibulotemporal Gelenks und/oder amyothrophische Lateralsklerose (ALS).

## Revendications

1. Composition pharmaceutique comprenant un composé de formule I ou de formule II ci-dessous sous forme de particules, ladite composition ayant une distribution monomodale des diamètres de particules de telle sorte que la valeur médiane du diamètre moyen en volume soit comprise dans l'intervalle de 350 à 700 nm, habituellement avec une quantité non supérieure à 10 % des particules ayant un diamètre volumique égal ou inférieur à 280 nm, et une quantité non inférieure à 90 % des particules ayant un diamètre volumique égal ou inférieur à 2000 nm : 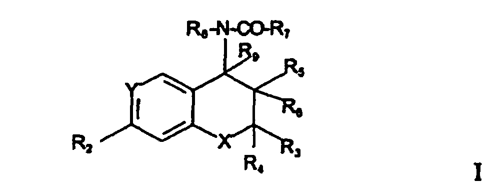 formule dans laquelle :
Y représente N et R₂ représente un atome d'hydrogène, ou bien Y représente un groupe C-R₁
où :
un des groupes R₁ et R₂ représente un atome d'hydrogène et l'autre est choisi dans le groupe consistant en un atome d'hydrogène, des groupes cycloalkyle en C₃ à C₈, alkyle en C₁ à C₆ facultativement interrompu par un atome d'oxygène ou substitué avec un substituant hydroxy, alkoxy en C₁ à C₆ ou aminocarbonyle substitué, (alkyle en C₁ à C₆)carbonyle, (alkoxy en C₁ à C₆)carbonyle, (alkyle en C₁ à C₆)carbonyloxy, alkoxy en C₁ à C₆, nitro, cyano, halogéno, trifluorométhyle, CF₃S, ou un groupe CF₃-A-, dans lequel A représente un groupe -CF₂-, -CO-, -CH₂- ou CH (OH) , trifluorométhoxy, alkylsulfinyle en C₁ à C₆, alkylsulfonyle en C₁ à C₆, alkoxysulfinyle en C₁ à C₆, alkoxysulfonyle en C₁ à C₆, aryle, hétéroaryle, arylcarbonyle, hétéroarylcarbonyle, arylsulfinyle, hétéroarylsulfinyle, arylsulfonyle, hétéroarylsulfonyle dans lequel n'importe quel groupement aromatique est facultativement substitué, (alkyle en C₁ à C₆)carbonylamino, (alkoxy en C₁ à C₆)carbonylamino, (alkyle en C₁ à C₆)thiocarbonyle, (alkoxy en C₁ à C₆)thiocarbonyle, (alkyle en C₁ à C₆)thiocarbonyloxy, 1-mercapto(alkyle en C₂ à C₇), formyle ou aminosulfinyle, aminosulfonyle ou aminocarbonyle, n'importe quel groupement amino étant facultativement substitué avec un ou deux groupes alkyle en C₁ à C₆, ou (alkyle en C₁ à C₆)sulfinylamino, (alkyle en C₁ à C₆)sulfonylamino, (alkoxy en C₁ à C₆)sulfinylamino ou (alkoxy en C₁ à C₆)sulfonylamino, ou éthylényle substitué en position terminale avec un substituant alkylcarbonyle, nitro ou cyano, ou -C(alkyle en C₁ à C₆)NOH ou -C(alkyle en C₁ à C₆)NNH₂, ou bien un des groupes R₁ et R₂ représente un groupe nitro, cyano ou (alkyle en C₁ à C₃)carbonyle et l'autre représente un groupe méthoxy ou amino facultativement substitué avec un ou deux substituants alkyle en C₁ à C₆ ou avec un substituant alcanoyle en C₂ à C₇ ;
un des groupes R₃ et R₄ représente un atome d'hydrogène ou un groupe alkyle en C₁ à C₄ et l'autre représente un groupe alkyle en C₁ à C₄, ou bien R₃ et R₄, conjointement, représentent un groupe polyméthylène en C₂ à C₅ ;
R₅ représente un groupe (alkyle en C₁ à C₆)carbonyloxy, benzoyloxy, ONO₂, benzyloxy, phényloxy ou alkoxy en C₁ à C₆ et R₆ et R₉ représentent des atomes d'hydrogène, ou bien R₅ représente un groupe hydroxy et R₆ représente un atome d'hydrogène ou un groupe alkyle en C₁ ou C₂ et R₉ représente un atome d'hydrogène ;
R₇ représente un groupe fluorophényle, R₈ représente un atome d'hydrogène ou un groupe alkyle en C₁ à C₆ ;
le groupe R₈-N-CO-R₇ étant en position *trans* par rapport au groupe R₅ ;
et X représente un atome d'oxygène ou un groupe NR₁₀ dans lequel R₁₀ représente un atome d'hydrogène ou un groupe alkyle en C₁ à C₆ ;
et 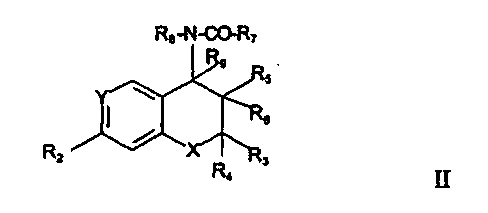 dans laquelle :
Y représente un atome de N et R₂ représente un atome d'hydrogène, ou bien Y représente un groupe C-R₁
où :
un des groupes R₁ et R₂ représente un atome d'hydrogène et l'autre est choisi dans le groupe consistant en un atome d'hydrogène, des groupes cycloalkyle en C₃ à C₈, alkyle en C₁ à C₆ facultativement interrompu par un atome d'oxygène ou substitué avec un substituant hydroxy, alkoxy en C₁ à C₆ ou aminocarbonyle substitué, (alkyle en C₁ à C₆)carbonyle, (alkoxy en C₁ à C₆)carbonyle, (alkyle en C₁ à C₆)carbonyloxy, alkoxy en C₁ à C₆, nitro, cyano, halogéno, trifluorométhyle, CF₃S ou un groupe CF₃-A- dans lequel A représente un groupe -CF₂-, -CO-, -CH₂-, CH(OH), SO₂, SO, CH₂-O ou CONH, ou un groupe CF₂H-A'- dans lequel A' représente un atome d'oxygène ou de soufre, un groupe SO, SO₂, CF₂ ou CFH ; trifluorométhoxy, alkylsulfinyle en C₁ à C₆, perfluoralkylsulfonyle en C₂ à C₆, alkylsulfonyle en C₁ à C₆, alkoxysulfinyle en C₁ à C₆, alkoxysulfonyle en C₁ à C₆, aryle, hétéroaryle, arylcarbonyle, hétéroarylcarbonyle, phosphono, arylcarbonyloxy, hétéroarylcarbonyloxy, arylsulfinyle, hétéroarylsulfinyle, arylsulfonyle, hétéroarylsulfonyle dans lequel n'importe quel groupement aromatique est facultativement substitué, (alkyle en C₁ à C₆)carbonylamino, (alkoxy en C₁ à C₆)carbonylamino, (alkyle en C₁ à C₆)thiocarbonyle, (alkoxy en C₁ à C₆)thiocarbonyle, (alkyle en C₁ à C₆)thiocarbonyloxy, 1-mercapto(alkyle en C₂ à C₇), formyle ou aminosulfinyle, aminosulfonyle ou aminocarbonyle, n'importe quel groupement amino étant facultativement substitué avec un ou deux groupes alkyle en C₁ à C₆, ou (alkyle en C₁ à C₆)sulfinylamino, (alkyle en C₁ à C₆)sulfonylamino, (alkoxy en C₁ à C₆)sulfinylamino ou (alkoxy en C₁ à C₆)sulfonylamino, ou éthylényle substitué en position terminale avec un substituant (alkyle en C₁ à C₆)carbonyle, nitro ou cyano, ou -C(alkyle en C₁ à C₆)NOH ou -C(alkyle en C₁ à C₆)NNH₂, ou bien un des groupes R₁ et R₂ représente un groupe nitro, cyano ou (alkyle en C₁ à C₃)carbonyle et l'autre représente un groupe méthoxy ou amino facultativement substitué avec un ou deux substituants alkyle en C₁ à C₆ ou avec un substituant alcanoyle en C₂ à C₇ ; ou bien R₁ et R₂, conjointement, représentent un groupe -(CH₂)₄- ou -CH=CH-CH=CH-, ou forment un noyau triazole ou oxadiazole facultativement substitué ;
un des groupes R₃ et R₄ représente un atome d'hydrogène ou un groupe alkyle en C₁ à C₄ et l'autre représente un groupe alkyle en C₁ à C₄, CF₃ ou CH₂X^{a} dans lequel X^{a} représente un groupe fluoro, chloro, bromo, iodo, alkoxy en C₁ à C₄, hydroxy, (alkyle en C₁ à C₄)carbonyloxy, -S(alkyle en C₁ à C₄), nitro, amino facultativement substitué avec ou deux groupes alkyle en C₁ à C₄ ; cyano ou (alkoxy en C₁ à C₄)carbonyle, ou bien R₃ et R₄, conjointement, représentent un groupe polyméthylène en C₂ à C₅ facultativement substitué avec un substituant alkyle en C₁ à C₄ ;
R₅ représente un groupe (alkyle en C₁ à C₆)carbonyloxy, benzoyloxy, ONO₂, benzyloxy, phényloxy ou alkoxy en C₁ à C₆ et R₆ et R₉ représentent des atomes d'hydrogène, ou bien R₅ représente un groupe hydroxy et R₆ représente un atome d'hydrogène ou un groupe alkyle en C₁ ou C₂ et R₉ représente un atome d'hydrogène ;
R₇ représente un groupe hétéroaryle ou phényle ; ces deux groupes étant facultativement substitués une ou plusieurs fois indépendamment avec un groupe ou atome choisi entre des groupes chloro, fluoro, bromo, iodo, nitro, amino facultativement substitué une ou deux fois avec un substituant alkyle en C₁ à C₄, cyano, azido, alkyle en C₁ à C₄, alkoxy en C₁ à C₄, trifluorométhoxy et trifluorométhyle ;
R₈ représente un atome d'hydrogène ; un groupe alkyle en C₁ à C₆, OR₉ ou NHCOR₁₀ dans lequel R₉ représente un atome d'hydrogène, un groupe alkyle en C₁ à C₆, formyle, alcanoyle en C₁ à C₆, aroyle ou aryl (alkyle en C₁ à C₆) et R₁₀ représente un atome d'hydrogène, un groupe alkyle en C₁ à C₆, alkoxy en C₁ à C₆, mono- ou di (alkyle en C₁ à C₆)amino, amino, amino-(alkyle en C₁ à C₆), hydroxyalkyle en C₁ à C₆, halogénalkyle en C₁ à C₆, (acyle en C₁ à C₆)oxy(alkyle en C₁ à C₆), (alkoxy en C₁ à C₆) carbonyl (alkyle en C₁ à C₆), aryle ou hétéroaryle ;
le groupe R₈-N-CO-R₇ étant en position *cis* par rapport au groupe R₅ ;
et X représente un atome d'oxygène ou un groupe NR₁₀ dans lequel R₁₀ représente un atome d'hydrogène ou un groupe alkyle en C₁ à C₆.

2. Composition suivant la revendication 1, dans laquelle le diamètre volumique médian est compris dans l'intervalle de 450 à 550 nm.

3. Composition suivant la revendication 2, dans laquelle 10 % des particules ont un diamètre volumique égal ou inférieur à 260 nm et 90 % des particules ont un diamètre volumique égal ou inférieur à 1450 nm.

4. Composition suivant l'une quelconque des revendications 1 à 3, qui peut être obtenue en soumettant à un broyage par voie humide les composés de formule I et de formule II sous forme d'une dispersion aqueuse.

5. Composition suivant la revendication 4, qui peut être obtenue par broyage par voie humide en utilisant un broyeur à billes à chambres multiples avec un passage unique ou une recirculation du produit entre les chambres.

6. Composition suivant la revendication 4 ou 5, qui peut être obtenue en utilisant des billes céramiques d'oxydes de terres rares comme milieu de broyage.

7. Composition suivant la revendication 5 ou 6, qui peut être obtenue en utilisant un broyeur à chambres multiples dans lequel les billes dans une chambre ont un diamètre inférieur à celui des billes dans les autres chambres.

8. Composition suivant la revendication 7, qui peut être obtenue en utilisant un intervalle de diamètres de billes de 0,4 mm à 1,25 mm dans chaque chambre.

9. Composition suivant l'une quelconque des revendications 1 à 8, comprenant un support soluble convenable comme excipient pour le séchage par pulvérisation.

10. Composition suivant la revendication 8, dans laquelle le support soluble est le mannitol.

11. Composition suivant l'une quelconque des revendications 1 à 10, comprenant un agent tensioactif pour maintenir les particules en suspension au cours du broyage et lors de la remise en suspension.

12. Composition suivant la revendication 11, dans laquelle l'agent tensioactif est le laurylsulfate de sodium.

13. Composition suivant l'une quelconque des revendications 1 à 12, comprenant un agent antiagglomération efficace après administration d'une formulation pharmaceutique à un patient.

14. Composition suivant la revendication 13, dans laquelle l'agent antiagglomération est l'hydroxypropylméthylcellulose.

15. Procédé pour la préparation d'une composition en particules dans laquelle la substance médicamenteuse est un composé de formule I ou II suivant la revendication 1, qui comprend les étapes consistant :
a) à soumettre à un broyage par voie humide la substance médicamenteuse sous forme d'une dispersion aqueuse dans un broyeur à billes à chambres multiples avec une circulation du produit entre les chambres, la dispersion aqueuse comprenant un support soluble convenable comme excipient pour le séchage par pulvérisation, un agent tensioactif pour maintenir les particules en suspension lors de la remise en suspension, et un agent antiagglomération efficace après administration d'une formulation pharmaceutique à un patient ; et
b) à sécher par pulvérisation la dispersion aqueuse après broyage.

16. Procédé suivant la revendication 15, dans lequel la dispersion aqueuse à broyer comprend environ 10 à 30 % en poids/poids d'un composé de formule I ou II, environ 4 à 15 % en poids/poids du support soluble, environ 0,1 à 0,4 % en poids/poids d'agent tensioactif et environ 1 % en poids/poids à 2 % en poids/poids de l'agent antiagglomération.

17. Utilisation d'une composition suivant l'une quelconque des revendications 1 à 14 pour la production d'un médicament destiné au traitement et/ou à la prophylaxie de l'anxiété, de la manie, de la dépression, d'attaques de panique et/ou de l'agression, de troubles associés à une hémorragie sous-arachnoïdienne ou à un choc neural, des effets associés au sevrage de substances entraînant une dépendance telles que la cocaïne, la nicotine, l'alcool et les benzodiazépines, de troubles pouvant être traités et/ou prévenus avec des agents anticonvulsivants, tels que l'épilepsie, y compris l'épilepsie post-traumatique, la maladie de Parkinson, la psychose, la migraine, l'ischémie cérébrale, la maladie d'Alzheimer et d'autres maladies dégénératives telles que la chorée de Huntington, de la schizophrénie, de troubles obsessionnels compulsifs (OCD), de déficiences neurologiques associées au SIDA, de troubles du sommeil (comprenant des troubles du rythme circadien, l'insomnie et la narcolepsie), des tics (par exemple le syndrome de Gilles de la Tourette), d'une lésion cérébrale traumatique, des bourdonnements d'oreille, des névralgies, notamment des névralgies du trijumeau, d'une douleur neuropathique, d'une douleur dentaire, d'une douleur provoquée par le cancer, d'une activité neuronale inappropriée ayant pour résultat des neurodysthésies dans des maladies telles que le diabète, la sclérose en plaques (MS) et une maladie neuronale motrice, des ataxies, de la rigidité musculaire (spasticité), d'un dysfonctionnement de l'articulation temporomandibulaire et/ou de la sclérose latérale amyotrophique (ALS).
